# EUROPEAN PATENT APPLICATION

(11) **EP 4 672 159 A1**
(43) Date of publication of application: **31.12.2025**
(21) Application number: 24763035.3
(22) Date of filing: 22.02.2024
(51) Int. Cl.: G06T 17/20

(54) **POINT CLOUD PROCESSING METHOD AND APPARATUS, DEVICE, AND STORAGE MEDIUM**

(30) Priority: 28.02.2023 CN 202310208654
(71) Applicant: Shining 3D Tech Co., Ltd., Hangzhou, Zhejiang 311258 (CN)
(72) Inventor: JIA, Yongjie, Hangzhou, Zhejiang 311258 (CN); JIANG, Tengfei, Hangzhou, Zhejiang 311258 (CN); ZHANG, Jian, Hangzhou, Zhejiang 311258 (CN); LIN, Zhongwei, Hangzhou, Zhejiang 311258 (CN); HUANG, Leijie, Hangzhou, Zhejiang 311258 (CN); LIU, Yu, Hangzhou, Zhejiang 311258 (CN); MA, Chao, Hangzhou, Zhejiang 311258 (CN)
(74) Representative: Hautier IP - MC/EP
(86) International application number: PCT/CN2024/078036
(87) International publication number: WO 2024/179352

(57) **Abstract**

The present application provides a point cloud processing method and apparatus, a device, and a storage medium. The method comprises: acquiring a current frame point cloud collected by a point cloud collection apparatus and a global model, wherein the global model is obtained by fusing historical frame point clouds collected by the point cloud collection apparatus, any three-dimensional point in the global model carries a weight, and the weight of any three-dimensional point represents the possibility that the three-dimensional point is a cluttered point or a target object; after the current frame point cloud is projected into the global model, projecting a plurality of light rays between the projected current frame point cloud and the point cloud collection apparatus to determine a target three-dimensional point through which the light rays pass in the global model; reducing the weight of the target three-dimensional point; and deleting the target three-dimensional point if the reduced weight of the target three-dimensional point satisfies a cluttered point deletion condition. Therefore, the cluttered point is accurately deleted.

## Description

### CROSS-REFERENCE TO RELATED APPLICATION

The present disclosure claims the priority to the Chinese patent application with the filing No. 202310208654.2 filed on February 28, 2023 with the Chinese Patent Office, and entitled "POINT CLOUD PROCESSING METHOD AND APPARATUS, DEVICE, AND STORAGE MEDIUM", which is incorporated herein by reference in entirety.

### TECHNICAL FIELD

The present disclosure relates to the technical field of point clouds, and particularly to a point cloud processing method and apparatus, an electronic device and a computer-readable storage medium.

### BACKGROUND ART

Three-dimensional scanning is primarily used for scanning spatial profiles, structures and colors of objects so as to obtain spatial coordinates, e.g., point cloud data, of surfaces of objects. Its significance lies in being capable of converting three-dimensional information about objects into digital signals that can be directly processed by computers, thus providing a quite convenient and rapid means for digitization of physical objects. However, during the three-dimensional scanning, invalid unwanted objects other than target objects may also be scanned, and form noise data (or noise points in point clouds), and these invalid noise data may affect subsequent processing; therefore, the scanned noise data need to be deleted.

### SUMMARY

In view of this, the present disclosure provides a point cloud processing method and apparatus, an electronic device and a computer-readable storage medium.

Specifically, the present disclosure is realized through technical solutions as follows.

According to the first aspect of embodiments of the present disclosure, a point cloud processing method is provided, including:
acquiring a point cloud of a current frame collected by a point cloud collection apparatus and a global model, where the global model is obtained by fusing point clouds of historical frames collected by the point cloud collection apparatus; and any three-dimensional point in the global model carries a weight, and the weight represents likelihood that the three-dimensional point is a noise point (cluttered point) or a target object;
casting, after projecting the point cloud of the current frame into the global model, several light rays between the projected point cloud of the current frame and the point cloud collection apparatus, so as to determine a target three-dimensional point in the global model through which the light rays pass;
reducing the weight of the target three-dimensional point; and
deleting the target three-dimensional point, if the reduced weight of the target three-dimensional point satisfies a noise-point deletion condition.

Optionally, the method further includes: determining initial weights of three-dimensional points in the point cloud of the current frame; and
after projecting the point cloud of the current frame into the global model, the method further includes:
updating the weights of the three-dimensional points of the point cloud of the current frame in a projection area of the global model using the initial weights of the three-dimensional points in the point cloud of the current frame.

Optionally, in the point cloud of the current frame, determining initial weights of three-dimensional points in the point cloud of the current frame includes:
acquiring, for each three-dimensional point in the point cloud of the current frame, a first adjustment coefficient based on collection information about the three-dimensional point; and
adjusting a reference weight using the first adjustment coefficient, so as to obtain the initial weight of the three-dimensional point, where the first adjustment coefficient is positively correlated with the initial weight.

Optionally, the collection information about each of the three-dimensional points includes at least one of: a distance between the three-dimensional point and an optimal depth of field of a point cloud collection apparatus, a distance between the three-dimensional point and a center of a field of view of the point cloud collection apparatus, a distance between the three-dimensional point and a light ray from which the three-dimensional point is interpolated among the light rays cast by the point cloud collection apparatus, or a difference between normal information about the three-dimensional point and normal information about a neighboring three-dimensional point thereof.

Herein, the first adjustment coefficient for the three-dimensional point is negatively correlated with the collection information about the three-dimensional point.

Optionally, determining initial weights of three-dimensional points in the point cloud of the current frame includes:
acquiring, for each three-dimensional point in the point cloud of the current frame, a first adjustment coefficient based on collection information about the three-dimensional point;
acquiring a second adjustment coefficient based on an object recognition result corresponding to the three-dimensional point, after performing object recognition processing on the point cloud of the current frame; and
adjusting a reference weight according to the first adjustment coefficient and the second adjustment coefficient, so as to obtain an initial weight of the three-dimensional point, where the second adjustment coefficient is positively correlated with the initial weight, and the second adjustment coefficient for a three-dimensional point where the object recognition result is a target object is greater than the second adjustment coefficient for a three-dimensional point where the object recognition result is a noise point.

Optionally, a starting point of the light rays is one of a first position and a second position, and an ending point of the light rays is the other of the first position and the second position.

Herein, the first position includes one of: a position where the projected point cloud of the current frame is located, or a result obtained by combining the position where the projected point cloud of the current frame is located with a preset error distance; and
the second position includes one of: a position where a lens is located when the point cloud collection apparatus collects the point cloud of the current frame, or a result obtained by combining the position where the projected point cloud of the current frame is located with a preset distance, where the preset distance is determined on the basis of a depth of field of the point cloud collection apparatus when collecting the point cloud of the current frame.

Optionally, the several light rays correspond one-to-one to several three-dimensional points in the point cloud of the current frame; and
reducing the weight of the target three-dimensional point includes:
determining a three-dimensional point in the point cloud of the current frame corresponding to a light ray passing through the target three-dimensional point, and reducing the weight of the target three-dimensional point using the weight of the three-dimensional point in the global model.

Optionally, a space where the global model is located is divided into several voxels; and
determining a target three-dimensional point in the updated global model through which the light rays pass includes:
detecting a target voxel through which the light rays pass, and determining the target three-dimensional point in the target voxel that intersects with the light rays.

Optionally, the method further includes:
performing weight accumulation, if at least one frame of point cloud subsequently collected by the point cloud collection apparatus includes a deleted target three-dimensional point, based on initial weights of the deleted target three-dimensional point in respective frames of point cloud; and
reconstructing the target three-dimensional point in the global model, when the accumulated weight of the deleted target three-dimensional point satisfies a predefined reconstruction condition.

Optionally, the three-dimensional points in the global model are displayed in a color gradient according to a descending or ascending order of weights, where display colors of the three-dimensional points indicated by different weights are different.

Optionally, the noise-point deletion condition indicates that a reduced weight of a target three-dimensional point is less than a weight threshold, and
the method further includes:
acquiring a weight threshold input by a user in an interactive interface; or
acquiring, in response to a scenario scanning instruction input by the user, a plurality of recommended values of the weight threshold from pre-stored data based on a scanning scenario indicated by the scenario scanning instruction, where the pre-stored data include the plurality of recommended values of the weight threshold under different scanning scenarios;
presenting the plurality of recommended values of the weight threshold in the interactive interface; and
determining, in response to a selection operation of the user, a selected value from the plurality of recommended values of the weight threshold.

According to the second aspect of embodiments of the present disclosure, a point cloud processing method is provided, including:
acquiring a global model collected by a point cloud collection apparatus, where any three-dimensional point in the global model carries a weight, and the weight of any three-dimensional point represents likelihood that the three-dimensional point is a noise point or a target object; and
deleting, if there is a target three-dimensional point in the global model that satisfies a noise-point deletion condition, the target three-dimensional point.

According to the third aspect of embodiments of the present disclosure, a point cloud processing apparatus is provided, including:
an acquisition module, configured to acquire a point cloud of a current frame collected by a point cloud collection apparatus and a global model, where the global model is obtained by fusing point clouds of historical frames collected by the point cloud collection apparatus; and any three-dimensional point in the global model carries a weight, and the weight of any three-dimensional point represents likelihood that the three-dimensional point is a noise point or a target object;
a target three-dimensional point determination module, configured to cast, after projecting the point cloud of the current frame into the global model, several light rays between the projected point cloud of the current frame and the point cloud collection apparatus, so as to determine a target three-dimensional point in the global model through which the light rays pass;
a weight reduction module, configured to reduce the weight of the target three-dimensional point; and
a target three-dimensional point deletion module, configured to delete the target three-dimensional point, if the reduced weight of the target three-dimensional point satisfies a noise-point deletion condition.

According to the fourth aspect of embodiments of the present disclosure, an electronic device is provided, including a memory, a processor and executable instructions stored on the memory and executable on the processor, where
the processor, when executing the executable instructions, implements the steps in the method according to any one of the first aspect.

According to the fifth aspect of embodiments of the present disclosure, a computer-readable storage medium is provided, storing computer instructions thereon, where the computer instructions, when executed by a processor, implements the steps in the method according to any one of the first aspect.

It should be understood that the above general description and the following detailed description are exemplary and illustrative only, and cannot limit the present disclosure.

### BRIEF DESCRIPTION OF DRAWINGS

Drawings herein are incorporated into the description and constitute a part of the description. They show embodiments complying with the present disclosure, and are used for explaining the principle of the present disclosure together with the description.
FIG. 1A is a schematic diagram showing a projection result of a frame of point cloud collected by a point cloud collection apparatus at time T onto a global model in an exemplary embodiment of the present disclosure.
FIG. 1B shows a scanning range of the point cloud collection apparatus within an oral cavity at time T in an exemplary embodiment of the present disclosure.
FIG. 2A is a schematic diagram showing a projection result of a frame of point cloud collected by the point cloud collection apparatus at time T+n onto a global model in an exemplary embodiment of the present disclosure.
FIG. 2B shows a scanning range of the point cloud collection apparatus within an oral cavity at time T+n in an exemplary embodiment of the present disclosure.
FIG. 3 is a schematic flowchart showing a point cloud processing method in an exemplary embodiment of the present disclosure.
FIG. 4 is an exemplary schematic diagram showing an electronic device in an exemplary embodiment of the present disclosure.
FIG. 5 is a structural schematic diagram showing a point cloud processing apparatus in an exemplary embodiment of the present disclosure.

### DETAILED DESCRIPTION OF EMBODIMENTS

Exemplary embodiments will be described in detail herein, examples of which are illustrated in the drawings. When drawings are involved in the following description, the same reference numerals in different drawings represent the same or similar elements unless otherwise indicated. Embodiments described in the following exemplary embodiments do not represent all embodiments consistent with the present disclosure. On the contrary, they are merely examples of an apparatus and a method consistent with the present disclosure in some aspects, as detailed in the appended claims.

The terms used in the present disclosure are merely for a purpose of describing particular embodiments, rather than limiting the present disclosure. Singular forms, "a/an", "the" and "this", used in the present disclosure and the appended claims are also intended to include plural forms, unless otherwise clearly indicated in the context. It also should be understood that the term "and/or" used herein refers to any or all possible combinations containing one or more associated items listed.

It should be understood that the terms such as first, second, and third may be used in the present disclosure for describing various kinds of information, but the information should not be limited to these terms. These terms are merely used for distinguishing information in a same category. For example, without departing from the scope of the present disclosure, first information also may be referred to as second information, similarly, the second information also may be referred to as the first information. Depending on the context, the word "if" as used herein may be construed as "when..." or "while..." or "in response to...".

Three-dimensional scanning is primarily used for scanning spatial profiles, structures and colors of objects, so as to obtain spatial coordinates, e.g., point cloud data, of surfaces of objects. Its significance lies in being capable of converting three-dimensional information about objects into digital signals that can be directly processed by computers, thus providing a quite convenient and rapid means for digitization of physical objects. However, during the three-dimensional scanning, invalid unwanted objects other than target objects may also be scanned, and form noise data (or noise points in point cloud data), and these invalid noise data may affect the subsequent processing process; therefore, the scanned noise data need to be deleted prior to subsequent processing.

For example, in the oral scanning field, a three-dimensional scanner can be used for scanning users' teeth, so as to construct three-dimensional dental models. The three-dimensional dental models can be used for occlusion detection, undercut treatment, etc. However, when scanning the teeth of the user using the three-dimensional scanner, besides valid data such as teeth and gums, invalid noise data such as lingual side, labial side, buccal side and intraoral medical equipment also may be scanned, and the invalid noise data may affect a subsequent tooth treatment process. Therefore, the invalid noise data need to be deleted.

Regarding the problems in the related art, embodiments of the present disclosure provide a point cloud processing method, which can acquire a point cloud of a current frame collected by a point cloud collection apparatus and a global model. The global model herein is obtained by fusing point clouds of historical frames collected by the point cloud collection apparatus; and any three-dimensional point in the global model carries a weight, and the weight of any three-dimensional point represents likelihood that the three-dimensional point is a noise point or a target object. In other words, the weight represents confidence of the three-dimensional point, where the higher the weight is, the higher the confidence is, and the higher the likelihood that the three-dimensional point is a target object is; and the lower the weight is, the lower the confidence is, and the higher the likelihood that the three-dimensional point is a noise point.

When the point cloud collection apparatus collects the point cloud of the current frame, it can be assumed that the collected point cloud of the current frame consists of valid data, while there are no other point cloud data between the point cloud of the current frame and the point cloud collection apparatus. In other words, there are theoretically no other objects between the point cloud collection apparatus and a detected object (i.e., the point cloud of the current frame). Based on the assumption, the point cloud of the current frame can be utilized to delete noise data in the global model. The point cloud of the current frame can be projected into the global model, and then several light rays are cast between the projected point cloud of the current frame and the point cloud collection apparatus, so as to determine a target three-dimensional point in the global model through which the light rays pass. The target three-dimensional point is likely a noise point between the point cloud of the current frame erroneously collected at a historical moment and the point cloud collection apparatus. In order to avoid erroneous deletion, the present embodiment reduces the weight of the target three-dimensional point. In turn, if the reduced weight of the target three-dimensional point satisfies a noise-point deletion condition, the target three-dimensional point is deleted, and if the reduced weight of the target three-dimensional point does not satisfy the noise-point deletion condition, the target three-dimensional point is not deleted. The point cloud collection apparatus collects a next frame of point cloud and repeats the above processing, so that noise point data can be accurately deleted upon multiple verifications, thereby improving accuracy of a deletion result.

The point cloud processing method provided in embodiments of the present disclosure can be executed by an electronic device. The electronic device includes, but is not limited to, a smartphone/mobile phone, a tablet computer, and a personal digital assistant (PDA), a laptop computer, a desktop computer, a media content player, a video game station/system, a virtual reality system, an augmented reality system, a wearable device (e.g., a watch, glasses, gloves, a headwear (e.g., a hat, a helmet, a virtual reality headset, an augmented reality headset, a head mounted device (HMD), and a headband), a pendant, an armband, a leg loop, shoes, and a waistcoat), a remote control, or any other devices with computer power.

Exemplarily, the electronic device includes a processor and a memory, where the memory stores executable instructions that can run on the processor, and the processor, when executing the executable instructions, implements the point cloud processing method provided by embodiments of the present disclosure. Exemplarily, the electronic device is integrated with a computer program product, and the electronic device, when executing the computer program product, implements the point cloud processing method provided by embodiments of the present disclosure.

Exemplarily, the electronic device can be provided with a point cloud collection apparatus; or the electronic device and the point cloud collection apparatus are independent from each other and are in communication connection with each other.

Herein, the point cloud collection apparatus includes, but is not limited to, a lidar, a millimeter wave radar, a binocular vision sensor, a structured light depth camera, or the like.

The lidar is configured to transmit a laser pulse sequence to a target scenario, then receive the laser pulse sequence reflected back from a target, and generate a three-dimensional point cloud based on the laser pulse sequence reflected back. In an example, the lidar can determine receiving time of the laser pulse sequence reflected back, for example, determining receiving time of the laser pulse sequence by detecting rising edge time and/or falling edge time of an electrical signal pulse. In this way, the lidar can calculate TOF (time of flight) using receiving time information and transmitting time of the laser pulse sequence, so as to determine a distance from a detected object to the lidar. The lidar is an autonomous light emitting sensor that does not rely on light source illumination, is less interfered by ambient light, and can work normally even in a closed environment without light, so as to subsequently generate a high-precision three-dimensional model, thus having wide applicability. The principle of point cloud collection of the millimeter wave radar is similar to that of the lidar, and is not repeated herein.

The binocular vision sensor acquires two images of a target scenario from different positions based on the parallax principle, and acquires three-dimensional geometric information by calculating position deviation between corresponding points in the two images, so as to generate a three-dimensional point cloud. The binocular vision sensor has low hardware requirements, correspondingly can also reduce costs, only needs an ordinary CMOS (complementary metal oxide semiconductor) camera, and can be used in both indoor and outdoor environments as long as light is appropriate; therefore, it also has certain applicability.

The structured light depth camera casts light rays with certain structural characteristics into a target scenario and then collects the same. Such light rays with a certain structure collect different image phase information due to different depth areas of a subject, and then convert it into depth information, so as to obtain a three-dimensional point cloud. The structured light depth camera is also an autonomous light emitting sensor that does not rely on light source illumination, is less interfered by ambient light, and can work normally even in a closed environment without light, so as to subsequently generate a high-precision three-dimensional model, thus having wide applicability.

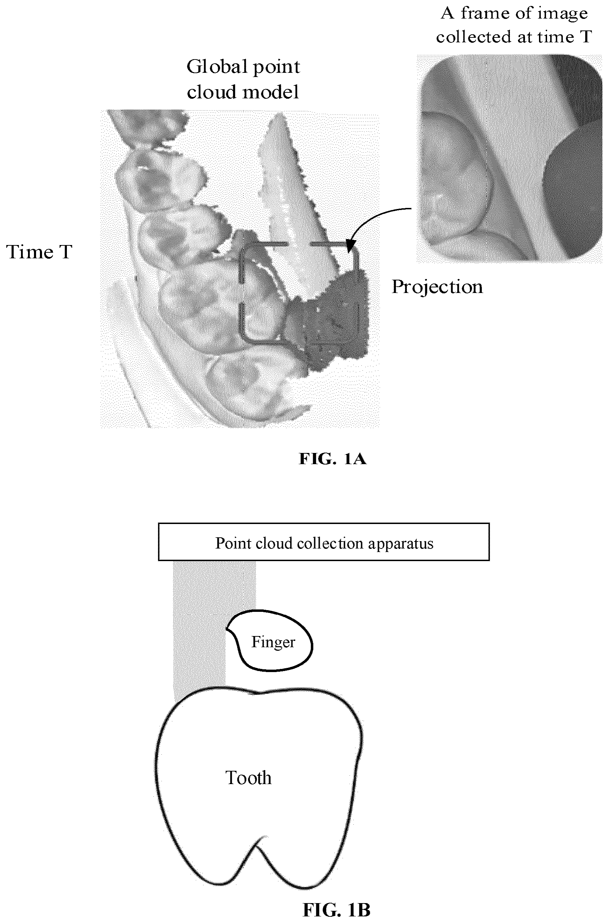

In an exemplary application scenario, for example, in the oral scanning field, the point cloud collection apparatus can perform scanning in an oral cavity of a user, so as to collect data related to teeth of the user, thereby establishing a three-dimensional model of teeth. As shown in FIG. 1A and FIG. 1B, FIG. 1A shows a projection result of a frame of point cloud collected by the point cloud collection apparatus at time T in the global model. For ease of understanding, this frame of point cloud is rendered image-wise in FIG. 1A, and the image may be a two-dimensional image or a depth image carrying depth information, which is not limited in any way in the present embodiment. FIG. 1B shows a scanning range of the point cloud collection apparatus within the oral cavity at time T. As can be seen from FIG. 1A and FIG. 1B, the point cloud collection apparatus scans a finger at time T, as a result, a collected frame of point cloud contains finger-related noise points. After the frame of point cloud collected by the point cloud collection apparatus at the time T is projected into the global model, noise data such as the finger appear in a projection area (square block in FIG. 1A) of the global model.

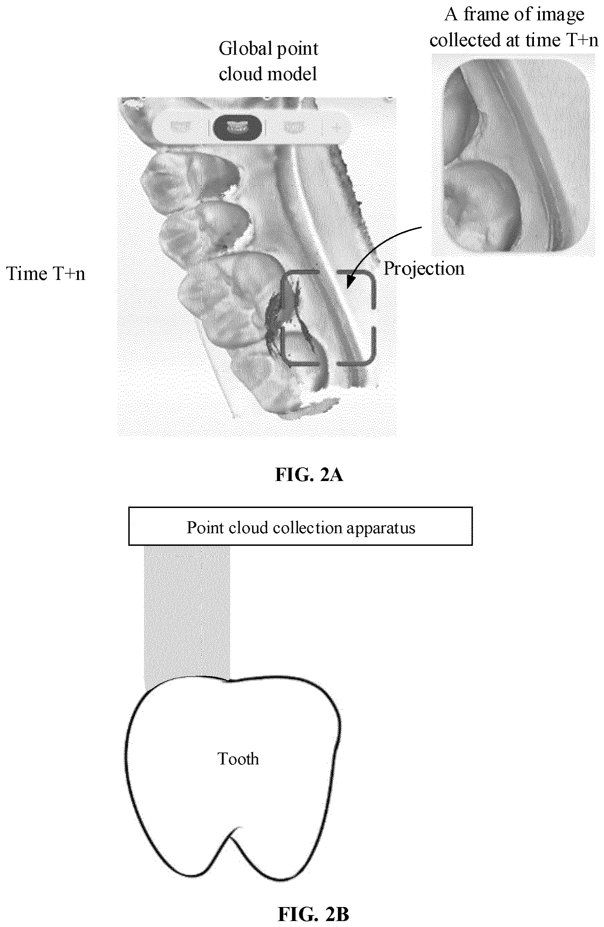

The point cloud collection apparatus continues scanning in the oral cavity of the user, and can apply the point cloud processing method provided by embodiments of the present disclosure c during the scanning, so as to delete noise data in real time. For example, at time T+N, the point cloud collection apparatus scans again a position intersecting with that scanned at time T. Referring to FIG. 2A and FIG. 2B, FIG. 2A shows a projection result of a frame of point cloud collected by the point cloud collection apparatus at time T+N in the global model, and this frame of point cloud is rendered image-wise in FIG. 2B. FIG. 2B shows a scanning range of the point cloud collection apparatus in the oral cavity at time T+n. As can be seen from FIG. 2A and FIG. 2B, the finger is not scanned by the point cloud collection apparatus at time T+n, where n is an integer greater than 0. Based on the assumption that there are theoretically no other objects between the point cloud collection apparatus and the detected object (i.e., the point cloud of the current frame), when the point cloud processing method provided by embodiments of the present disclosure is applied, weights of the finger-related noise points collected at time T in the global model can be reduced; when the same position is scanned multiple times, the weights of the finger-related noise points can be reduced multiple times , and finally, when the reduced weights satisfy the noise-point deletion condition, the finger-related noise points collected at a historical moment in the global model are deleted, thus realizing accurate deletion of noise points upon multiple verifications.

Hereinafter, the point cloud processing method provided in embodiments of the present disclosure is exemplarily described.

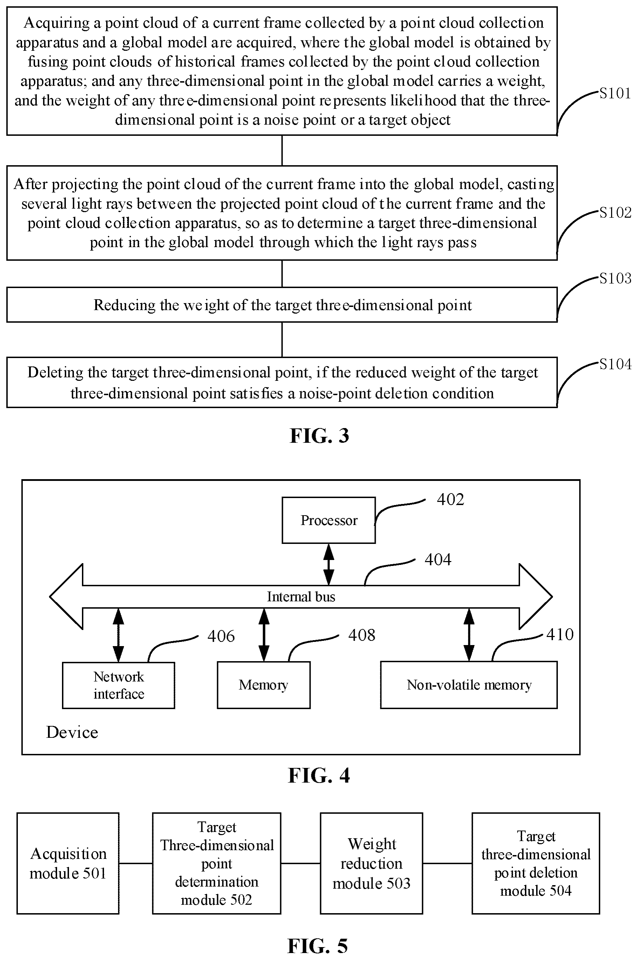

It should be noted that the point cloud collection apparatus in embodiments of the present disclosure needs to scan the same position in a detection scenario multiple times, and in this way, the noise data scanned at the same position at a historical moment can be filtered out and deleted based on a scanning result at current time. Referring to FIG. 3, FIG. 3 shows a schematic flowchart of a point cloud processing method. The method can be executed by an electronic device. The method includes:
At S101, a point cloud of a current frame collected by a point cloud collection apparatus and a global model are acquired, where the global model is obtained by fusing point clouds of historical frames collected by the point cloud collection apparatus; and any three-dimensional point in the global model carries a weight, and the weight of any three-dimensional point represents likelihood that the three-dimensional point is a noise point or a target object.
At S102, after the point cloud of the current frame is projected into the global model, several light rays are cast between the projected point cloud of the current frame and the point cloud collection apparatus, so as to determine a target three-dimensional point in the global model through which the light rays pass.
At S103, the weight of the target three-dimensional point is reduced.
At S104, the target three-dimensional point is deleted, if the reduced weight of the target three-dimensional point satisfies a noise-point deletion condition.

In the present embodiment, it can be assumed that the collected point cloud of the current frame consists of valid data, and there are no other point cloud data between the point cloud of the current frame and the point cloud collection apparatus. In other words, there are theoretically no other objects between the point cloud collection apparatus and a detected object (i.e., the point cloud of the current frame). Based on the assumption, the point cloud of the current frame can be utilized to delete noise data in the global model between the point cloud of the current frame collected at a historical moment and the point cloud collection apparatus. The point cloud of the current frame can be projected into the global model, and then several light rays are cast between the projected point cloud of the current frame and the point cloud collection apparatus, so as to determine the target three-dimensional point in the global model through which the light rays pass. The target three-dimensional point is likely a noise point between the point cloud of the current frame erroneously collected at a historical moment and the point cloud collection apparatus. In order to avoid erroneous deletion, the present embodiment reduces the weight of the target three-dimensional point, and the weight can be reduced multiple times based on the case where the target three-dimensional point is passed through by the light rays multiple times until the reduced weight of the target three-dimensional point satisfies the noise-point deletion condition, and then the target three-dimensional point is deleted, so that noise point data can be accurately deleted upon multiple verifications, thereby improving the accuracy of a deletion result.

Exemplarily, the noise-point deletion condition indicates that a weight of a three-dimensional point is less than a preset threshold, where the preset threshold can be specifically set according to an actual application scenario.

Exemplarily, the global model is obtained by fusing point clouds of historical frames collected by the point cloud collection apparatus. In an example, the global model includes several (a plurality of) three-dimensional points. In another example, besides several three-dimensional points, the global model can further include triangle information formed by three-dimensional connection; that is, the global model may be a mesh model.

In some embodiments, after acquiring the point cloud of the current frame collected by the point cloud collection apparatus, the electronic device further needs to determine initial weights of three-dimensional points in the point cloud of the current frame.

In a possible implementation, it may be assumed that all three-dimensional points in the point cloud of the current frame collected by the point cloud collection apparatus are valid data, that is, preset initial weights can be assigned to the three-dimensional points in the point cloud of the current frame, and the initial weights of all three-dimensional points in the point cloud of the current frame are the same.

In another possible implementation, the initial weights of the three-dimensional points can be determined based on degrees of reliability of depth information about the three-dimensional points in the point cloud of the current frame, where the higher the degree of reliability is, the greater the initial weight is. Herein, the degrees of reliability of the depth information about the three-dimensional points can be reflected by collection information about the three-dimensional points. Exemplarily, for each three-dimensional point in the point cloud of the current frame, the electronic device can acquire a first adjustment coefficient based on the collection information about the three-dimensional point, and then adjust a reference weight using the first adjustment coefficient, so as to obtain the initial weight of the three-dimensional point. Herein, the first adjustment coefficient is positively correlated with the initial weight, that is, the larger the first adjustment coefficient is, the larger the initial weight is, and vice versa. In an example, the initial weight of the three-dimensional point is equal to a product of the first adjustment coefficient and the reference weight. In another example, the initial weight of the three-dimensional point is equal to a sum of the first adjustment coefficient and the reference weight. In the present embodiment, a specific adjustment manner of adjusting the reference weight using the first adjustment coefficient is not limited in any way, and can be specifically set according to an actual application scenario.

Exemplarily, the collection information about each three-dimensional point includes at least one of the following: a distance between the three-dimensional point and an optimal depth of field of the point cloud collection apparatus, a distance between the three-dimensional point and a center of a field of view of the point cloud collection apparatus, a distance between the three-dimensional point and a light ray from which the three-dimensional point is interpolated among the light rays cast by the point cloud collection apparatus, or a difference between normal information about the three-dimensional point and normal information about a neighboring three-dimensional point thereof, but is not limited thereto. In an example, if the point cloud collection apparatus is a structured light camera, the cast light rays are structured light; and if the point cloud collection apparatus is a lidar, the cast light rays are laser rays.

Based on practical demonstration, it can be determined that: (1) the depth of the three-dimensional point collected within a depth of field of the point cloud collection apparatus is more reliable; therefore, the smaller the distance between the three-dimensional point and the optimal depth of field is, the larger the first adjustment coefficient is, and vice versa; (2) the depth of the three-dimensional point collected at the center of the field of view of the point cloud collection apparatus is more reliable; therefore, the smaller the distance between the three-dimensional point and the center of the field of view is, the larger the first adjustment coefficient is, and vice versa; (3) the closer the three-dimensional point is to the light ray from which the three-dimensional point is interpolated among the light rays cast by the point cloud collection apparatus, the more reliable the depth of the three-dimensional point is; therefore, the smaller the distance between the three-dimensional point and the light ray from which the three-dimensional point is interpolated among the light rays cast by the point cloud collection apparatus is, the larger the first adjustment coefficient is, and vice versa; and (4) the depth of the three-dimensional point with a smaller degree of depth jump is more reliable; therefore, the smaller the difference between the normal information about the three-dimensional point and the normal information about a neighboring three-dimensional point thereof is, the larger the first adjustment coefficient is, and vice versa.

That is to say, the first adjustment coefficient for the three-dimensional point is negatively correlated with any one of the following: the distance between the three-dimensional point and the depth of field, the distance between the three-dimensional point and the center of the field of view, the distance between the three-dimensional point and a position where the light ray is cast, and the difference between the normal information about the three-dimensional point and the normal information about the neighboring three-dimensional point thereof.

In another possible implementation, in addition to considering the degree of reliability of the depth information about the three-dimensional point in the point cloud of the current frame, a degree of significance of the three-dimensional point can be further considered, and the higher the degree of significance of the three-dimensional point is, the higher the initial weight is. Herein, the degree of significance of the three-dimensional point can be reflected by an object recognition result of the three-dimensional point, and if the three-dimensional point is recognized as a target object, such as tooth and gum, then it is indicated that the three-dimensional point is relatively significant; and if the three-dimensional point is recognized as a noise point, then it is indicated that the three-dimensional point is insignificant.

Exemplarily, for each three-dimensional point in the point cloud of the current frame, the electronic device can acquire the first adjustment coefficient based on the collection information about the three-dimensional point; after performing object recognition processing on the point cloud of the current frame, acquire a second adjustment coefficient based on an object recognition result corresponding to the three-dimensional point; and next, adjust the reference weight according to the first adjustment coefficient and the second adjustment coefficient, so as to obtain the initial weight of the three-dimensional point. Herein, the second adjustment coefficient is positively correlated with the initial weight, and the second adjustment coefficient for a three-dimensional point where the object recognition result is a target object is greater than the second adjustment coefficient for a three-dimensional point where the object recognition result is a noise point.

In an example, the initial weight of the three-dimensional points may be a product of the first adjustment coefficient, the second adjustment coefficient and the reference weight. In another example, the initial weight of the three-dimensional points also may be a sum of the first adjustment coefficient, the second adjustment coefficient and the reference weight. In the present embodiment, a specific adjustment manner of adjusting the reference weight using the first adjustment coefficient and the second adjustment coefficient is not limited in any way, and can be specifically set according to an actual application scenario.

Herein, one or more kinds of target objects may be included, and the second adjustment coefficients that indicate three-dimensional points of different kinds of target objects can be the same or different, and can be specifically set according to an actual application scenario. In an example, assuming that in the dental scanning field, the target objects include teeth and gums, the second adjustment coefficient for the three-dimensional points where the object recognition result is tooth can be set to be larger than the second adjustment coefficient for the three-dimensional points where the object recognition result is gum.

In an example, taking that the initial weight of the three-dimensional point may be the product of the first adjustment coefficient, the second adjustment coefficient and the reference weight as an example, a value of the initial weight is between 0 and 1, a value of the reference weight is 1, and values of the first adjustment coefficient and the second adjustment coefficient are between 0 and 1. It is assumed that the target objects include teeth and gums in the field of dental scanning, the second adjustment coefficient for three-dimensional points which are teeth can be set to be 1, the second adjustment coefficient for three-dimensional points which are gums can be set to be 0.5, and the second adjustment coefficient for three-dimensional points which are noise points can be set to be 0. Certainly, other values can also be set, which is not limited in any way in the present embodiment.

Herein, when performing the object recognition processing on the point cloud of the current frame, a pre-trained object recognition model can be utilized to process the point cloud of the current frame, so as to obtain object recognition results corresponding to the three-dimensional points in the point cloud of the current frame. Exemplarily, the object recognition model can be obtained by performing supervised training based on several frames of point clouds and object labels thereof.

In some embodiments, the global model is obtained by fusing point clouds of historical frames collected by the point cloud collection apparatus. That is to say, whenever the point cloud collection apparatus collects a frame of point cloud, this frame of point cloud can be fused into the global model. Herein, a collection position of the point cloud of the current frame and collection positions of the point clouds of the historical frames have an overlapping part, that is, the point cloud of the current frame and the global model contain three-dimensional points collected by the point cloud collection apparatus from the same position, and only in this way, noise points in the global model can be filtered out based on the point cloud of the current frame.

Any three-dimensional point in the global model carries a weight. In an example, the larger the weight of a three-dimensional point in the global model is, the higher the likelihood that the three-dimensional point is a target object and the more reliable the three-dimensional point is; and the smaller the weight is, the higher the likelihood that the three-dimensional point is a noise point and the less reliable the three-dimensional point is.

After projecting the point cloud of the current frame into the global model, the electronic device can update the weights of the three-dimensional points of the point cloud of the current frame in a projection area of the global model using the initial weights of the three-dimensional points in the point cloud of the current frame. Exemplarily, after a certain three-dimensional point in the point cloud of the current frame is projected to a certain projection position in the global model, if there is no three-dimensional point in the projection position, the initial weight of the three-dimensional point in the point cloud of the current frame can be taken as the weight of the three-dimensional point in the projection position. If there is already a three-dimensional point in the projection position, the initial weight of the three-dimensional point in the point cloud of the current frame can be accumulated with the weight of the three-dimensional point in the projection position, and the accumulated weight is taken as an updated weight of the three-dimensional point in the projection position.

After the point cloud of the current frame is projected into the global model, the electronic device can cast several light rays between the projected point cloud of the current frame and the point cloud collection apparatus, so as to determine a target three-dimensional point in the global model through which the light rays pass. The electronic device herein can cast several light rays between the projected point cloud of the current frame and the point cloud collection apparatus; and can also cast several light rays between the projection area corresponding to the projected point cloud of the current frame and the point cloud collection apparatus, which is not limited in any way in the present embodiment.

Exemplarily, a starting point of the light rays is one of a first position and a second position, and an ending point of the light rays is the other of the first position and the second position. Herein, the first position may be a position where the projected point cloud of the current frame is located; alternatively, in order to avoid a situation that the projected point cloud of the current frame is erroneously processed, the first position may be a result obtained by combining the position where the projected point cloud of the current frame is located with a preset error distance. A specific value of the preset error distance can be specifically set according to an actual application scenario, which is not limited in any way in the present embodiment.

The second position may be a position where a lens is located when the point cloud collection apparatus collects the point cloud of the current frame; alternatively, in order to improve casting efficiency of light rays, the second position may be a result obtained by combining the position where the projected point cloud of the current frame is located with a preset distance, where the preset distance is determined on the basis of the depth of field of the point cloud collection apparatus when collecting the point cloud of the current frame, so as to avoid or reduce the casting of light rays to an area where no three-dimensional point is collected, thereby improving the light casting efficiency.

In an example, the light rays take the three-dimensional points of the projected point cloud of the current frame as starting points, and extend in a direction towards a position where the point cloud collection apparatus is located. In another example, the starting points of the light rays may be results obtained by adding a preset error distance to coordinates of the three-dimensional points of the projected point cloud of the current frame, and then extend towards a direction of the position where the point cloud collection apparatus is located.

In some embodiments, after several light rays are cast between the projected point cloud of the current frame and the point cloud collection apparatus, the electronic device can determine a target three-dimensional point in the global model through which the light rays pass. Based on the assumption that there are theoretically no other objects between the point cloud collection apparatus and the detected object (i.e., the point cloud of the current frame), the target three-dimensional point in the global model through which the light rays pass is likely a noise point between the point cloud of the current frame erroneously collected at a historical moment and the point cloud collection apparatus.

In some possible embodiments, the electronic device can exhaustively check whether any three-dimensional point in the global model intersects with any of the several light rays, so as to determine the target three-dimensional points in the global model through which the light rays pass.

In another possible implementation, a space where the global model is located can be divided into several voxels. Voxel can be understood as the smallest unit of digital data in three-dimensional spatial segmentation, conceptually similar to the smallest unit in a two-dimensional space-pixel, where the pixel is used on image data of a two-dimensional computer image. The voxel uses a constant scalar quantity or a vector to represent a three-dimensional region. In order to reduce computational complexity and improve computational efficiency, in the process of an electronic device determining the target three-dimensional point, the electronic device can first detect a target voxel among the several voxels through which the light rays pass, and then further determine the target three-dimensional point in the target voxel that intersects with the light rays. Because an area represented by a single voxel is larger than a single three-dimensional point, the method of first determining the target voxel and then determining the target three-dimensional point can significantly reduce a computational amount and improve computational efficiency compared with the above exhaustive method.

In some embodiments, after determining the target three-dimensional points in the global model through which the light rays pass, the electronic device can reduce the weights of the target three-dimensional points, and further delete the target three-dimensional points when the reduced weights of the target three-dimensional points satisfy the noise-point deletion condition. In order to avoid erroneous deletion, in the present embodiment, detection of noise points is a continuous process. If a certain three-dimensional point is a noise point, the weight of the three-dimensional point can be reduced multiple times through multiple scanning processes until a modified weight of the three-dimensional point satisfies the noise-point deletion condition, thus realizing accurate deletion of the noise point upon multiple verifications, and improving the accuracy of the deletion result. Exemplarily, the noise-point deletion condition indicates that the weight of the three-dimensional point is less than the preset threshold.

In a possible embodiment, in a case where a smaller weight indicates higher likelihood that the three-dimensional point is a noise point, the electronic device can subtract a preset value from the weight of the target three-dimensional point. Further, if the reduced weight of the target three-dimensional point is less than the weight threshold, the electronic device can delete the target three-dimensional point.

Exemplarily, at least one of the weight threshold and the preset value (i.e., reduction proportion) can be set by the user according to actual needs, a specific numerical value can be input by the user according to his/her own experience, and some recommended values can also be given for the user to choose according to actual application scenarios.

In an example, the electronic device is provided with an input means (e.g., an interactive interface, a keyboard, a mouse, etc.), and the electronic device can receive the weight threshold and the preset value input by the user in the input means.

In another example, in response to a scenario scanning instruction input by the user, the electronic device acquires a plurality of recommended values of the weight threshold and/or a plurality of recommended values of the preset value from the pre-stored data based on a scanning scenario indicated by the scenario scanning instruction, where the pre-stored data include the plurality of recommended values of the weight threshold and/or the plurality of recommended values of the preset value under different scanning scenarios; then the electronic device displays the plurality of recommended values of the weight threshold and/or the plurality of recommended values of the preset value in the interactive interface for the user to adjust; and further in response to a selection operation of the user, determines a selected value from the plurality of recommended values of the weight threshold, and/or determines a selected value from a plurality of candidate values of the preset value.

Herein, the numerical values of the weight threshold and the preset value will affect removal efficiency of the noise points and removal accuracy of the noise points.

In an example, if the weight threshold is set relatively high and the preset value is set relatively low, the weight of the noise point cannot be reduced to be lower than the weight threshold at one time because the preset value is small, then multiple comparisons are required to delete the noise point. For example, if the preset value is 0.3, the weight threshold is 0.1, and the weight of the noise point is 1, then whenever it is determined that the noise point is passed through by the light rays, the weight of the noise point is reduced by 0.3, and the weight needs to be reduced at least three times before the noise point can be deleted. The process of comprising multiple times and deletion is beneficial to improving the deletion accuracy of the noise point, but the deletion efficiency of the noise point is reduced to some extent.

In another example, for example, if the weight threshold is set relatively low and the preset value is set relatively high, the weight of the noise point can be reduced to be less than the weight threshold through at one or a fewer times as the preset value is relatively large, and then the noise point can be deleted directly through a single or fewer comparison processes, thereby improving the deletion efficiency of the noise point.

Therefore, the user can set the weight threshold and the preset value that satisfy his/her own requirements according to his/her own needs on removal efficiency of the noise points and removal accuracy of the noise points.

In another possible embodiment, the starting points or the ending points of the light rays may be three-dimensional points in the point cloud of the current frame. That is to say, each of the several light rays cast in the above uniquely corresponds to one of the several three-dimensional points in the point cloud of the current frame. Thus, in order to further precisely modify the weight of the target three-dimensional point, the electronic device can determine a three-dimensional point in the point cloud of the current frame corresponding to a light ray passing through the target three-dimensional point, and reduce the weight of the target three-dimensional point using the weight of the three-dimensional point in the global model. In the present embodiment, the weight of the target three-dimensional point is adaptively reduced according to the weight of the three-dimensional point in the point cloud of the current frame corresponding to the light ray, thus precisely modifying the weights of the target three-dimensional points through which different light rays pass.

In an example, in a case where a larger weight indicates higher likelihood that a three-dimensional point is a target object, and a smaller weight indicates higher likelihood that a three-dimensional point is a noise point, after determining the three-dimensional point in the point cloud of the current frame corresponding to the light ray passing through the target three-dimensional point, the electronic device can reduce the weight of the target three-dimensional point using the weight of the three-dimensional point in the global model. The larger the weight of the three-dimensional point is, the more the weight of the target three-dimensional point is reduced. For example, the weight of the three-dimensional point in the global model can be subtracted from the weight of the target three-dimensional point. For another example, a numerical value corresponding to the weight of the three-dimensional point in the global model can be subtracted from the weight of the target three-dimensional point, and the numerical value is positively correlated with the weight of the three-dimensional point. Further, if the reduced weight of the target three-dimensional point is less than the weight threshold, the electronic device can delete the target three-dimensional point. Herein, the weight threshold can be set by the user according to actual needs, a specific numerical value can be input by the user according to his/her own experience, and some recommended values can also be given for the user to choose according to actual application scenarios.

In other words, the larger the weight of the three-dimensional point in the point cloud of the current frame corresponding to the light rays passing through the target three-dimensional point is, the higher the likelihood that the three-dimensional point is a target object, and then the more the weight of the target three-dimensional point is reduced. On the contrary, the smaller the weight of the three-dimensional point in the point cloud of the current frame corresponding to the light ray passing through the target three-dimensional point is, the lower the likelihood that the three-dimensional point is a target object, and then the less the weight of the target three-dimensional point is reduced.

In some embodiments, different three-dimensional points in the global model have different weights, and then display colors of the different three-dimensional points are also different, so that the user can learn in real time the magnitude of likelihood that different three-dimensional points are target objects or noise points. Exemplarily, the three-dimensional points in the global model can be displayed in a color gradient according to a descending or ascending order of weights, where display colors of the three-dimensional points indicated by different weights are different.

In some embodiments, the user can select a locked area in the global model according to actual needs, where the locked area indicates that any three-dimensional point located in the locked area is valid data that the user wants to obtain, and there is no need to modify the weight or delete. After the point cloud of the current frame is projected into the global model, if the projected point cloud of the current frame is located in the locked area, subsequent operations are not required.

In some embodiments, considering that erroneous deletion may occur if a deleted target three-dimensional point is not a noise point but is erroneously determined as a noise point, if at least one frame of point cloud subsequently collected by the point cloud collection apparatus contains a deleted target three-dimensional point, the electronic device can perform weight accumulation based on initial weights of the deleted target three-dimensional point in various frames of point cloud; and then reconstruct the target three-dimensional point in the global model when the accumulated weight of the deleted target three-dimensional point satisfies a predefined reconstruction condition. In other words, in a case where the deleted target three-dimensional point is included when the point cloud collection apparatus collects and scans, it indicates high likelihood that the deleted target three-dimensional point is erroneously deleted, and then restoration of the deleted target three-dimensional point can be realized through the weight accumulation.

In an example, the electronic device can reconstruct the target three-dimensional point in the global model when the accumulated weight of the deleted target three-dimensional point is greater than the weight threshold. by means of initial weight accumulation, restoration of the deleted target three-dimensional point in the global model is realized through multiple verifications.

Herein, it is not hard to understand that the solutions described in various embodiments in the above can be combined without conflict, and embodiments of the present disclosure are not enumerated.

In some embodiments, a point cloud processing method includes: acquiring a global model collected by a point cloud collection apparatus, where any three-dimensional point in the global model carries a weight, and the weight of any three-dimensional point represents likelihood that the three-dimensional point is a noise point or a target object; and if there is a target three-dimensional point in the global model that satisfies a noise-point deletion condition, the target three-dimensional point is deleted.

Herein, the above weight can be adjusted according to the above embodiments, Ai intelligent systems (for example, in a case where a three-dimensional point is recognized as a non-target object, reducing the weight of the three-dimensional point), historical clinical case data of patients, scanning scenarios, for example, whether scanning a dental model or a patient's oral cavity, and user-set rules.

FIG. 4 is a structural schematic diagram of a device provided in an exemplary embodiment. Referring to FIG. 4, on a hardware level, the device includes a processor 402, an internal bus 404, a network interface 406, a memory 408 and a non-volatile memory 410, and certainly may further include hardware required by other services. One or more embodiments of the present description can be implemented in a software-based manner, for example, the processor 402 reads a corresponding computer program from the non-volatile memory 410 into the memory 408 and then runs the same. Certainly, in addition to the software implementation, one or more embodiments of the present description do not exclude other implementations, for example, logic devices or a combination of software and hardware. That is to say, execution subjects of the following processing procedure are not limited to various logic units, and may also be hardware or logic devices.

Referring to FIG. 5, a point cloud processing apparatus can be applied to the device as shown in FIG. 4, so as to implement technical solutions of the present description. Herein, the point cloud processing apparatus may include:
an acquisition module 501, configured to a point cloud of a current frame collected by a point cloud collection apparatus and a global model, where the global model is obtained by fusing point clouds of historical frames collected by the point cloud collection apparatus; and any three-dimensional point in the global model carries a weight, and the weight of any three-dimensional point represents likelihood that the three-dimensional point is a noise point or a target object;
a target three-dimensional point determination module 502, configured to, after the point cloud of the current frame is projected into the global model, cast several light rays between the projected point cloud of the current frame and the point cloud collection apparatus, so as to determine a target three-dimensional point in the global model through which the light rays pass;
a weight reduction module 503, configured to reduce the weight of the target three-dimensional point; and
a target three-dimensional point deletion module 504, configured to delete the target three-dimensional point, if the reduced weight of the target three-dimensional point satisfies a noise-point deletion condition.

In some embodiments, behind the acquisition module 501, the point cloud processing apparatus further includes a weight determination module, configured to determine initial weights of three-dimensional points in the point cloud of the current frame. The weight determination module is further configured to, after the point cloud of the current frame is projected into the global model, update weights of three-dimensional points of the point cloud of the current frame in a projection area of the global model using the initial weights of the three-dimensional points in the point cloud of the current frame.

In some embodiments, the weight determination module is specifically configured to, for each three-dimensional point in the point cloud of the current frame, acquire a first adjustment coefficient based on the collection information about the three-dimensional point; and
adjust a reference weight using the first adjustment coefficient, so as to obtain the initial weight of the three-dimensional point, where the first adjustment coefficient is positively correlated with the initial weight.

The collection information about each three-dimensional point includes at least one of the following: a distance between the three-dimensional point and an optimal depth of field of the point cloud collection apparatus, a distance between the three-dimensional point and a center of a field of view of the point cloud collection apparatus, a distance between the three-dimensional point and a light ray from which the three-dimensional point is interpolated among the light rays cast by the point cloud collection apparatus, or a difference between normal information about the three-dimensional point and normal information about a neighboring three-dimensional point thereof.

Herein, the first adjustment coefficient for the three-dimensional point is negatively correlated with the collection information about the three-dimensional point.

In some embodiments, the weight determination module is specifically configured to: for each three-dimensional point in the point cloud of the current frame, acquire the first adjustment coefficient based on the collection information about the three-dimensional point;
acquire a second adjustment coefficient based on an object recognition result corresponding to the three-dimensional point, after object recognition processing is performed on the point cloud of the current frame; and
adjust the reference weight according to the first adjustment coefficient and the second adjustment coefficient, so as to obtain the initial weight of the three-dimensional point. Herein, the second adjustment coefficient is positively correlated with the initial weight, and the second adjustment coefficient for a three-dimensional point where the object recognition result is a target object is greater than the second adjustment coefficient for a three-dimensional point where the object recognition result is a noise point.

In some embodiments, a starting point of the light rays is one of a first position and a second position, and an ending point of the light rays is the other of the first position and the second position.

Herein, the first position includes one of: a position where the projected point cloud of the current frame is located, or a result obtained by combining the position where the projected point cloud of the current frame is located with a preset error distance.

The second position includes one of: a position where a lens is located when the point cloud collection apparatus collects the point cloud of the current frame, or a result obtained by combining the position where the projected point cloud of the current frame is located with a preset distance, where the preset distance is determined on the basis of the depth of field of the point cloud collection apparatus when collecting the point cloud of the current frame.

In some embodiments, each of the several light rays uniquely corresponds to one of the several three-dimensional points in the point cloud of the current frame; and the weight reduction module 503 is specifically configured to: determine a three-dimensional point in the point cloud of the current frame corresponding to a light ray passing through the target three-dimensional point, and reduce the weight of the target three-dimensional point using the weight of the three-dimensional point in the global model.

In some embodiments, a space where the global model is located can be divided into several voxels; and the target three-dimensional point determination module 502 is specifically configured to detect a target voxel through which the light rays pass, and further determine the target three-dimensional point in the target voxel that intersects with the light rays.

In some embodiments, the point cloud processing apparatus further includes a reconstruction module, configured to, if at least one frame of point cloud subsequently collected by the point cloud collection apparatus includes a deleted target three-dimensional point, perform weight accumulation based on initial weights of the deleted target three-dimensional point in various frames of point cloud; and reconstruct the target three-dimensional point in the global model when the accumulated weight of the deleted target three-dimensional point satisfies a predefined reconstruction condition.

In some embodiments, the three-dimensional points in the global model are displayed in a color gradient according to a descending or ascending order of weights, where display colors of the three-dimensional points indicated by different weights are different.

In some embodiments, the noise-point deletion condition indicates that a reduced weight of a three-dimensional point is less than a weight threshold. The apparatus further includes: an interactive module, configured to acquire a weight threshold input by the user in an interactive interface; or in response to a scenario scanning instruction input by the user, acquire a plurality of recommended values of the weight threshold from pre-stored data based on a scanning scenario indicated by the scenario scanning instruction, where the pre-stored data include the plurality of recommended values of the weight threshold under different scanning scenarios; display the plurality of recommended values of the weight threshold in the interactive interface; and in response to a selection operation of the user, determine a selected value from the plurality of recommended values of the weight threshold.

For implementation processes of functions and effects of various modules in the above apparatus, reference can be made to implementation processes of corresponding steps in the above method, which will not be repeated herein.

Correspondingly, embodiments of the present disclosure further provide an electronic device, including a memory, a processor and executable instructions stored on the memory and executable on the processor;
Herein, the processor, when executing the executable instructions, implements the steps of the above method.

Exemplarily, the processor includes, but is not limited to, a central processing unit (CPU), a graphics processing unit (GPU), a digital signal processor (DSP), an application specific integrated circuit (ASIC), a field-programmable gate array (FPGA), etc.

Exemplarily, the memory may include at least one type of storage medium, and the storage medium includes a flash memory, a hard disk, a multimedia card, and a card-type memory (such as SD or DX memory), a random access memory (RAM), a static RAM, (SRAM), a read-only memory (ROM), an electrically erasable programmable ROM (EEPROM), a programmable ROM (PROM), a magnetic memory, a magnetic disk, an optical disk, etc.

Correspondingly, embodiments of the present disclosure further provide a computer program product, including a computer program, where the computer program, when executed by a processor, implements the above method.

In exemplary embodiments, further provided is a non-transitory computer-readable storage medium including instructions, for example a memory including instructions, where the instructions are executable by the processor of the apparatus so as to implement the above method. For example, the non-transitory computer-readable storage medium may be a ROM, a random access memory (RAM), a CD-ROM, a magnetic tape, a floppy disk, an optical data storage device, etc.

A non-transitory computer-readable storage medium, when instructions in the storage medium are executed by a processor of a terminal, enables the terminal to execute the above method.

Embodiments of subject matter and functional operations described in the present description can be implemented in digital electronic circuitry, tangibly embodied computer software or firmware, computer hardware including the structures disclosed in the present description and their structural equivalents, or a combination of one or more of them. Embodiments of the subject matters described in the present description can be implemented as one or more computer programs, i.e., one or more modules of computer program instructions encoded on a tangible, non-transitory program carrier to be executed by a data processing apparatus or to control the operation of the data processing apparatus. Alternatively or additionally, program instructions can be encoded on an artificially generated propagated signal, e.g., a machine-generated electrical, optical, or electromagnetic signal that is generated to encode the information and transmit the information to suitable receiver apparatus for execution by a data processing apparatus. The computer storage medium can be a machine-readable storage device, a machine-readable storage substrate, a random or serial access memory device, or a combination of one or more of them.

The processes and logic flows described in the present description can be executed by one or more programmable computers executing one or more computer programs, so as to perform corresponding functions by operating on input data and generating output. The processes and logic flows can also be executed by application specific logic circuitry, e.g., an FPGA (Field Programmable Gate Array) or an ASIC (Application Specific Integrated Circuit), and apparatus can also be implemented as application specific logic circuitry.

A computer suitable for executing a computer program includes, for example, a general-purpose and/or application specific microprocessor, or any other type of central processing unit. Typically, the central processing unit will receive instructions and data from a read only memory and/or a random access memory. The basic components of a computer include a central processing unit for implementing or executing instructions and one or more memory devices for storing instructions and data. Typically, the computer will also include one or more mass storage devices (e.g., magnetic disks, magneto-optical disks or optical disks) for data storage. Alternatively, the computer can be operatively coupled to such mass storage devices to either receive data from them, transfer data to them, or both. However, the computer does not necessarily have such a device. In addition, the computer can be embedded in another device, such as a mobile telephone, a personal digital assistant (PDA), a mobile audio or video player, a game console, a global positioning system (GPS) receiver, or a portable storage device such as a universal serial bus (USB) flash drive, just to name a few examples.

Computer-readable media suitable for storing computer program instructions and data include all forms of non-volatile memories, media and memory devices, including by way of example semiconductor memory devices, (for example, EPROM, EEPROM and flash memory devices), magnetic disks (for example, internal hard drives or removable disks), magneto-optical disks; and CD ROM and DVD-ROM disks. The processors and the memories can be supplemented by or incorporated in application-specific logic circuitry.

While the present description contains numerous specific implementation details, these should not be construed to limit the scope of any invention or claimed invention, but rather to primarily describe features of specific embodiments of particular inventions. Certain features described in multiple embodiments within the present description can also be implemented in combination in a single embodiment. In another aspect, various features described in a single embodiment can also be implemented separately in multiple embodiments or in any suitable sub-combination. Furthermore, although features may function in certain combinations as described above and are even initially claimed as such, one or more features from a claimed combination can in some cases be eliminated from the combination, and the claimed combination may be directed to a sub-combination or variation of a sub-combination.

Similarly, while operations are depicted in the drawings in a particular order, this should not be construed to require that the operations be performed in the particular order shown or serially, or that all illustrated operations be performed, to achieve desired results. In some cases, multitasking and parallel processing may be advantageous. Furthermore, separation of various system modules and components in the above embodiments should not be understood as requiring such separation in all embodiments, and it should be understood that the described program components and systems generally can be integrated together in a single software product or packaged into multiple software products.

Thus, specific embodiments of the subject matters have been described. Other embodiments are within the scope of the appended claims. In certain situations, actions defined in the claims can be performed in a different order and still achieve the desired results. Furthermore, the processes depicted in the drawings do not necessarily require the particular order shown, or sequential order, to achieve desired results. In some implementations, multitasking and parallel processing may be advantageous.

The above are merely for preferred embodiments of the present disclosure, rather than limiting the present disclosure, and any amendments, equivalent replacements, improvements and so on, made within the spirit and principle of the present disclosure, should be covered within the scope of protection of the present disclosure.

### INDUSTRIAL APPLICABILITY

The point cloud processing method and apparatus, the device and the storage medium in the present disclosure can accurately delete the noise point data upon multiple verifications, thus improving the accuracy of the deletion result, so that the erroneous deletion can be avoided, thereby demonstrating strong industrial applicability.

## Claims

1. A point cloud processing method, **characterized by** comprising steps of:
acquiring a point cloud of a current frame collected by a point cloud collection apparatus and a global model, wherein the global model is obtained by fusing point clouds of historical frames collected by the point cloud collection apparatus; and any three-dimensional point in the global model carries a weight, and the weight of any three-dimensional point represents likelihood that the three-dimensional point is a noise point or a target object;
casting, after projecting the point cloud of the current frame into the global model, several light rays between the projected point cloud of the current frame and the point cloud collection apparatus, so as to determine a target three-dimensional point in the global model through which the light rays pass;
reducing the weight of the target three-dimensional point; and
deleting the target three-dimensional point, if the reduced weight of the target three-dimensional point satisfies a noise-point deletion condition.

2. The method according to claim 1, further comprising a step of:
determining initial weights of three-dimensional points in the point cloud of the current frame; and
after the step of projecting the point cloud of the current frame into the global model, further comprising a step of:
updating the weights of the three-dimensional points of the point cloud of the current frame in a projection area of the global model using the initial weights of the three-dimensional points in the point cloud of the current frame.

3. The method according to claim 2, wherein in the point cloud of the current frame, the step of determining initial weights of three-dimensional points in the point cloud of the current frame comprises:
acquiring, for each three-dimensional point in the point cloud of the current frame, a first adjustment coefficient based on collection information about the three-dimensional point; and
adjusting a reference weight using the first adjustment coefficient, so as to obtain the initial weight of the three-dimensional point, wherein the first adjustment coefficient is positively correlated with the initial weight.

4. The method according to claim 3, wherein the collection information about each of the three-dimensional points comprises at least one of: a distance between the three-dimensional point and an optimal depth of field of a point cloud collection apparatus, a distance between the three-dimensional point and a center of a field of view of the point cloud collection apparatus, a distance between the three-dimensional point and a light ray from which the three-dimensional point is interpolated among the light rays cast by the point cloud collection apparatus, or a difference between normal information about the three-dimensional point and normal information about a neighboring three-dimensional point thereof, wherein
the first adjustment coefficient for the three-dimensional point is negatively correlated with the collection information about the three-dimensional point.

5. The method according to claim 1, wherein the step of determining initial weights of three-dimensional points in the point cloud of the current frame comprises:
acquiring, for each three-dimensional point in the point cloud of the current frame, a first adjustment coefficient based on collection information about the three-dimensional point;
acquiring a second adjustment coefficient based on an object recognition result corresponding to the three-dimensional point, after performing object recognition processing on the point cloud of the current frame; and
adjusting a reference weight according to the first adjustment coefficient and the second adjustment coefficient, so as to obtain an initial weight of the three-dimensional point, wherein the second adjustment coefficient is positively correlated with the initial weight, and the second adjustment coefficient for a three-dimensional point where the object recognition result is a target object is greater than the second adjustment coefficient for a three-dimensional point where the object recognition result is a noise point.

6. The method according to claim 1, wherein a starting point of the light rays is one of a first position and a second position, and an ending point of the light rays is the other of the first position and the second position, wherein
the first position comprises one of: a position where the projected point cloud of the current frame is located, or a result obtained by combining the position where the projected point cloud of the current frame is located with a preset error distance; and
the second position includes one of: a position where a lens is located when the point cloud collection apparatus collects the point cloud of the current frame, or a result obtained by combining the position where the projected point cloud of the current frame is located with a preset distance, wherein the preset distance is determined on the basis of a depth of field of the point cloud collection apparatus when collecting the point cloud of the current frame.

7. The method according to any one of claims 1 to 6, wherein the several light rays correspond one-to-one to several three-dimensional points in the point cloud of the current frame; and
the step of reducing the weight of the target three-dimensional point comprises:
determining a three-dimensional point in the point cloud of the current frame corresponding to a light ray passing through the target three-dimensional point, and reducing the weight of the target three-dimensional point using the weight of the three-dimensional point in the global model.

8. The method according to any one of claims 1 to 6, wherein a space where the global model is located is divided into several voxels; and
the step of determining a target three-dimensional point in an updated global model through which the light rays pass comprises:
detecting a target voxel through which the light rays pass, and determining the target three-dimensional point in the target voxel that intersects with the light rays.

9. The method according to any one of claims 1 to 6, further comprising:
performing weight accumulation, if at least one frame of point cloud subsequently collected by the point cloud collection apparatus comprises a deleted target three-dimensional point, based on initial weights of the deleted target three-dimensional point in respective frames of point cloud; and
reconstructing the target three-dimensional point in the global model, when an accumulated weight of the deleted target three-dimensional point satisfies a predefined reconstruction condition.

10. The method according to any one of claims 1 to 6, wherein the three-dimensional points in the global model are displayed in a color gradient according to a descending or ascending order of weights, wherein display colors of the three-dimensional points indicated by different weights are different.

11. The method according to any one of claims 1 to 6, wherein the noise-point deletion condition indicates that a reduced weight of a target three-dimensional point is less than a weight threshold, and
the method further comprises steps of:
acquiring a weight threshold input by a user in an interactive interface; or
acquiring, in response to a scenario scanning instruction input by the user, a plurality of recommended values of the weight threshold from pre-stored data based on a scanning scenario indicated by the scenario scanning instruction, wherein the pre-stored data comprise the plurality of recommended values of the weight threshold under different scanning scenarios;
presenting the plurality of recommended values of the weight threshold in the interactive interface; and
determining, in response to a selection operation of the user, a selected value from the plurality of recommended values of the weight threshold.

12. A point cloud processing method, **characterized by** comprising steps of:
acquiring a global model collected by a point cloud collection apparatus, wherein any three-dimensional point in the global model carries a weight, and the weight of any three-dimensional point represents likelihood that the three-dimensional point is a noise point or a target object; and
deleting, if there is a target three-dimensional point in the global model that satisfies a noise-point deletion condition, the target three-dimensional point.

13. A point cloud processing apparatus, **characterized by** comprising:
an acquisition module, configured to acquire a point cloud of a current frame collected by a point cloud collection apparatus and a global model, wherein the global model is obtained by fusing point clouds of historical frames collected by the point cloud collection apparatus; and any three-dimensional point in the global model carries a weight, and the weight of any three-dimensional point represents likelihood that the three-dimensional point is a noise point or a target object;
a target three-dimensional point determination module, configured to cast, after projecting the point cloud of the current frame into the global model, several light rays between the projected point cloud of the current frame and the point cloud collection apparatus, so as to determine a target three-dimensional point in the global model through which the light rays pass;
a weight reduction module, configured to reduce the weight of the target three-dimensional point; and
a target three-dimensional point deletion module, configured to delete the target three-dimensional point, if the reduced weight of the target three-dimensional point satisfies a noise-point deletion condition.

14. An electronic device, **characterized by** comprising a memory, a processor and executable instructions stored on the memory and executable on the processor, wherein
the processor, when executing the executable instructions, implements the steps in the method according to any one of claims 1 to 12.

15. A computer-readable storage medium, **characterized by** storing computer instructions thereon, wherein the computer instructions, when executed by a processor, implements the steps in the method according to any one of claims 1 to 12.
